(19) Europäisches Patentamt
European Patent Office
Office européen des brevets

(11) **EP 3 144 669 A1**

(12) **EUROPEAN PATENT APPLICATION**

(43) Date of publication:
**22.03.2017 Bulletin 2017/12**

(51) Int Cl.:
***G01N 27/12*** *(2006.01)*     *G01N 33/497* *(2006.01)*

(21) Application number: **16189400.1**

(22) Date of filing: **19.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **17.09.2015 US 201562219742 P**

(71) Applicant: **IDT Europe GmbH**
**01109 Dresden (DE)**

(72) Inventors:
• **Bergstrom, John**
**Santa Cruz, CA 95062 (US)**
• **Georgi, Michael**
**01705 Freital (DE)**
• **Oh, Joohye**
**04428 Youngsan-gu (KR)**

(74) Representative: **Lippert Stachow Patentanwälte Rechtsanwälte**
**Partnerschaft mbB**
**Krenkelstrasse 3**
**01309 Dresden (DE)**

(54) **A SINGLE GAS SENSOR FOR SENSING DIFFERENT GASES AND A METHOD USING THE GAS SENSOR**

(57) The invention relates to a single gas sensor for sensing different gases and to a method for sensing different gases using the single gas sensor. The object of the inventions to find a sense element configuration that reduces the thermal budget and decreases response time will be solved by a single gas sensor for sensing different gases comprising a MOX resistor as sensing element, a heater, and a signal conditioning circuitry, whereas said signal conditioning circuitry comprises a heater control unit connected to the heater, a first measurement unit for measuring the heater resistance, a second measurement unit for measuring the MOX-resistor resistance and a state machine as a system controller. The object will also be solved by setting the heater and the MOX resistor to a second temperature, then measuring the MOX resistance at time intervals predetermined via the state machine for the second temperature, then processing measured data and identifying a gas composition and calculating a concentration of different gases according to a determined temperature-resistance profile.

Fig. 3

EP 3 144 669 A1

**Description**

The invention relates to a single gas sensor for sensing different gases.

The invention relates also to a method for sensing different gases using the single gas sensor.

The 'Internet of Things' (IoT) is based on the ability to gather information about the environment of the consumer and using that information to enhance the lifestyle of the consumer. The generation of inertial sensors and pressure sensors have reached maturity - sensor fusion has enabled location based services and gesture recognition and monitoring of physical activity. The next phase will be gathering information about the environment of the consumer and providing vital information to the consumer. The metal oxide (MOX) chemiresistor gas sensor technology has been used for industrial applications since 1970s. There is interest to apply this technology to consumer handheld applications. The MOX technology has been established for the industrial applications but application to consumer applications requires a new generation of sensors and signal conditioning ASICs. Because gas sensors are only working well under certain conditions because they are highly influenced by humidity, temperature and effectiveness of chemisorption's reaction towards target gas. Generally, there are four types of gas sensors i.e. semiconductor metal-oxide (MOX), conducting polymer, optical and gravimetric sensor. Among these four types of gas sensors, MOX gas sensors are the most commonly used in industry due to their high sensitivity and fast response time in gas sensing. Besides, they also have the characteristics of easy to use, low maintenance cost, simple electronic interface, ability to detect large number of gases, and most importantly they are low cost. Now the challenging task is to apply the MOX technology successfully to handheld platforms.

Background

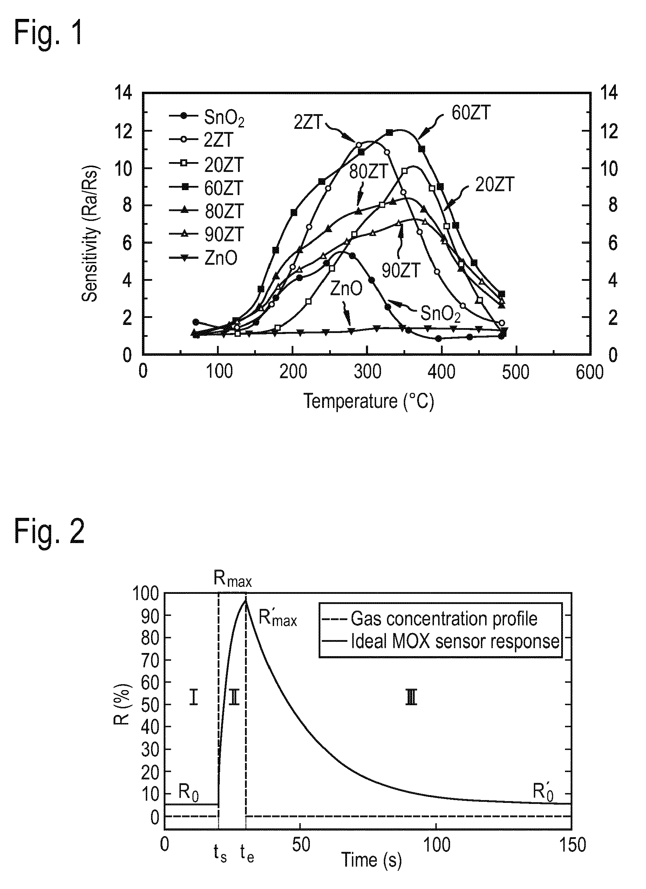

The interest for gas sensing for mobile platforms is focused on indoor and outdoor air quality. Indoor air quality is typically carbon monoxide (CO), breathe alcohol, and volatile organic compounds (VOC) especially formaldehyde. MOX based sensors have been proven for detecting and measuring CO, breathe alcohol and VOC in the atmosphere. Since 1962 it has been known that absorption or desorption of a gas on the surface of a metal oxide changes the conductivity of the material, this phenomenon being first demonstrated using zinc oxide thin film layers. The sensitivity of a surface to a gas can be as low as parts per billion (ppb). It is highly desirable that metal oxide semiconductor sensors have a large surface area, so as to adsorb as much of the target analyte as possible on the surface, giving a stronger and more measurable response (especially at low concentrations). So, especially for MOX sensors, their fast response in detecting gas is extremely advantageous, but they require a very long recovery time after the exposure of the target gas. It takes around 15 to 70 seconds to recover back to the baseline level after the target gas is removed. The baseline level is a threshold of the sensor output signal during absence of target gas. The reason for long recovery time of MOX gas sensing is due to its working principles. During the presence of target gas, the molecules of target gas contact with the detecting surface of MOX gas sensor, and then the chemisorption takes place at the detecting surface of the metal-oxide, this chemisorption reaction will result in chemical electrode effects and changes in sensor resistance value. Through the measurement of sensor resistance values, the target gas concentration percentage can be calculated as the chemisorption's reaction is proportional to gas concentration percentage, and the changes of sensor resistance is proportional to the chemisorption's reaction. Therefore, during the removal of target gas, there are still some gas molecules remain at the detecting surface of MOX gas sensor, and it takes a long period of time for all these molecules to desorb from the detecting surface of MOX gas sensor. As a consequence, it causes the long recovery time of MOX gas sensor after the exposure of the target gas. Figure 2 shows the response of a MOX gas sensor when exposed to a gas plume (during dashed region), and it take approximately a while to recover back to its baseline.

Furthermore, advances in fabrication methods have enabled the production of low-cost sensors with improved sensitivity and reliability compared to those formed using previous methods. Production costs are kept low due to the simplicity of metal oxide semiconductor sensor devices. Their ability to be produced quickly and on a large scale with easily controllable processes makes them a desirable technology to exploit. MOX technology can be based for example on SnO sensing systems with dopants and catalysts which maximize sensitivity and reduce cross sensitivity.

However, the application of MOX technology to handheld platforms will require a change in the operation mode for reduction in footprint and power reduction. There are some paradigm shifts that are required: the MOX sensor system requires a heater to take the MOX sensing material to the sensing temperature. For example, figure 1 shows the temperature dependence of the sensitivity of different gases of different MOX sensors, like $SnO_2$ and ZnO.

As stated above, chemical interactions must take place between the sensor and atmospheric gases, so response speed depends on MOX chemistry, particle size, sense element temperature, flow rate, gas composition, and gas concentration.

Figure 2 shows an ideal response of a MOX sensor (represented by the solid line) when excited with a step gas concentration (represented by a dashed line). The curve shows three phases of a measurement: (I) baseline, (II) gas measurement, and (III) recovery phase.

These phases can be expressed by the following equation for a transient response in gas concentration:

$$R(t) = \begin{cases} R_0 & , t < t_s \\ R_0 + (R_{max} - R_0)\left(1 - e^{\frac{-(t-t_s)}{\tau_r}}\right) & , t_s < t < t_e \\ R_0' + (R_{max}' - R_0')e^{\frac{-(t-t_s-\Delta t)}{\tau_d}} & , t_e < t \end{cases} \qquad (\text{eq. 1})$$

In equation 1 $\tau_r$ and $\tau_d$ are the time constants for the rise and recovery phases respectively, $t_s$ and $t_e$ represent the starting and ending times of the step excitation, $R_0$ and $R_0'$ are the sensor response level before and after the stimulus, $R_{max}$ is the saturation level, and $R_{max}'$ is the maximum response level during the gas measurement phase. Notice that $R_{max}'$ is usually lower than $R_{max}$ for short input pulses, as depicted in figure 2. $\tau_r$ and $\tau_d$ are dependent on MOX structure, MOX temperature and chemistry, but are determined empirically.

Gas sensing systems for industrial (non-mobile platforms) operate the sensor in an analog steady state mode and the power required is typically between 25mW and 75mW. Power consumption at these levels is unacceptable for a handheld platform. Inertial sensors in smart phones typically use 0.8mW full power and have the capability to be put into a 'sleep mode' that reduces power consumption to less than 20μW.

So, as stated above, sensors in mobile platforms or for penetration and acceptance in the smart phone and tablet market need to fulfill special requirements according to their size and power consumption. It has been clearly stated that the requirement that the MOX sense material is heated is a significant challenge. The challenge is especially the dynamic nature of the metal oxide compositions, because the metal oxide - gas composition reaction does not stop.

Therefore, it is a challenge to find a MOX material that can detect multiple gases. Furthermore, it is an object of the inventions to find a sense element configuration that reduces the thermal budget and decreases response time. And for heating the sensing element a heater is necessary which will minimize power consumption.

The object of the invention will be solve by a single gas sensor for sensing different gases comprising a MOX resistor as sensing element, a heater, and a signal conditioning circuitry, whereas said signal conditioning circuitry comprises a heater control unit connected to the heater, a first measurement unit for measuring the heater resistance, a second measurement unit for measuring the MOX-resistor resistance and a state machine as a system controller. One may consider this single gas sensor as an emulation of an array of gas sensors with a single MOX sense element and a signal conditioning ASIC. The MOX resistor has a surface of a metal oxide film which interacts with the target gas, generally through surface adsorbed oxygen ions, which results in a change in charge carrier concentration of the surface material. This change in charge carrier concentration serves to alter the conductivity (or resistivity) of the material of the MOX resistor. An n-type semiconductor is one where the majority charge carriers are electrons, and upon interaction with a reducing gas an increase in conductivity occurs. Conversely, an oxidizing gas serves to deplete the sensing layer of charge carrying electrons, resulting in a decrease in conductivity. A p-type semiconductor is a material that conducts with positive holes being the majority charge carriers; hence, the opposite effects are observed with the material and showing an increase in conductivity in the presence of an oxidizing gas (where the gas has increased the number of positive holes). A resistance increase with a reducing gas is observed, where the negative charge introduced in to the material reduces the positive (hole) charge carrier concentration. The heater is configured such that it heats the sensor element to a certain temperature. Thereby, the heater is connected to a heater control unit which is controllable or operable connected to the state machine. The heater control unit may be part of the signal conditioning circuitry. The signal conditioning circuitry also comprises a first measurement unit for measuring the heater resistance, a second measurement unit for measuring the MOX-resistor resistance and a state machine as a system controller. The first measurement unit is used for measuring the heater resistance in order to determine the thermal energy that is submitted to the MOX resistor by the heater. The second measurement unit is used for measuring the MOX-resistor resistance in dependence of the temperature and of the present gases that interact with the surface of the metal oxide film of the MOX resistors. The state machine works as a system controller which pretends to set the temperature to a pre-determined value in order to perform the inventive method for detecting and calculating concentrations based on the measured results.

In an embodiment of the invention the signal conditioning circuitry is an Application Specific Integrated Circuit (ASIC). An ASIC is an integrated circuit (IC) customized for a particular use, rather than intended for general-purpose use. Therefore, it is possible to reduce chip area and precisely adapt its functionality to a special task, here for controlling and setting the temperature and signal processing of the single gas sensor application.

In another embodiment the heater control unit is controllable connected to the heater for adjusting a pre-defined temperature regime to the heater. This is necessary to perform the inventive method, whereas the MOX resistor is set to a first temperature and afterwards to a second temperature which is higher than the first temperature. Advantageous, the temperature regime is adjustable form 100°C to 550°C, whereas the measurements for each temperature step is performed at a sampling rate in excess of 1000/s. So, in one second 1000 temperature values are measured by the signal conditioning circuit.

To use the inventive sensor for mobile platforms, the sensor need to be small and has to have a small power consumption. Therefore, the MOX resistor and the heater are stacked on a silicon die on a thin membrane or on a ceramic substrate with a low thermal mass. This is achieved for example by using anodic alumina for reducing the substrate thickness. Other methods for reducing the substrate thickness are possible, for example etching techniques, and so on. So, the sensor element has a small mass, a good thermal isolation and consists of a material that allows a rapid change in temperature, for example 96% Alumina, glass, and micromachined silicon with Si oxide or Si nitride diaphragms.

In one embodiment the state machine is controllable connected to the heater control unit, to the first measurement unit for measuring the heater resistance, and to the second measurement unit for measuring the MOX-resistor resistance.

The object of the invention will also be solved by a method comprising the following steps:

- setting the heater and the MOX resistor to a first temperature;
- measuring the MOX resistance at time intervals pre-determined via the state machine for the first temperature;
- setting the heater and the MOX resistor to a second temperature;
- measuring the MOX resistance at time intervals pre-determined via the state machine for the second temperature;
- processing measured data and identifying a gas composition and calculating a concentration of different gases according to a determined temperature-resistance profile. These profiles are determined from MOX material properties and the gas properties.

The temperature of the sense element, thus the MOX resistor and the heater are set to a first temperature T1. The MOX resistor is measured at intervals that can be adjusted via the state machine that resides in the control circuitry. The temperature is then set to a second temperature and the measurements are repeated as stated above. As a result of the measurement procedure one will get the temperature settings, delay times and the sensor output results of the MOX resistor, forming a matrix of data that indicate gas composition and concentration for carbon monoxide, breath alcohol and/or volatile organic compounds (VOC). The temperatures and delay times will be pre-determined for specific use cases.

The second temperature is higher than the first temperature, whereas the temperature vary from 100°C to 550°C.

Furthermore, it is advantageous when the time intervals for measuring are set to a sampling rate in excess of 1000/s. Because it is necessary to determine the transient response for selectivity.

In one embodiment the method captures the transient response which varies independently for different gases. Thereby, the measurement is divided into three phases: a base line measurement, a gas measurement and a recovery measurement. The base line measurement is performed without a presence of any gas. So, the baseline level is a threshold of the sensor output signal during absence of a target gas. The gas measurement is performed with the presence of a gas composition. During the presence of target gas, the molecules of target gas contact with the detecting surface of MOX gas sensor, and then the chemisorptions takes place at the detecting surface of the metal-oxide, this chemisorptions reaction will result in chemical electrode effects and changes in sensor resistance value. Through the measurement of sensor resistance values, the target gas concentration percentage can be calculated as the chemisorption's reaction is proportional to gas concentration percentage, and the changes of sensor resistance is proportional to the chemisorption's reaction. The recovery measurement is performed by waiting and detecting until the $R_0$ level is reached again. During the removal of target gas, there are still some gas molecules remain at the detecting surface of MOX gas sensor, and it takes a long period of time for all these molecules to desorb from the detecting surface of MOX gas sensor. As a consequence, it causes the long recovery time of MOX gas sensor after the exposure of the target gas.

The inventive presented single gas sensor is used for indicating a gas composition and concentration for carbon monoxide, breathe alcohol and/or volatile organic compounds (VOC). The best fit from the temperature points will identify composition and concentration.

The invention will be explained in more detail using an exemplary embodiment.

The appended drawings show

Fig. 1     Temperature dependence of the sensitivity of different gases;

Fig. 2     An ideal response of a MOX sensor when excited with a step gas concentration;

Fig. 3     The inventive sensor configuration.

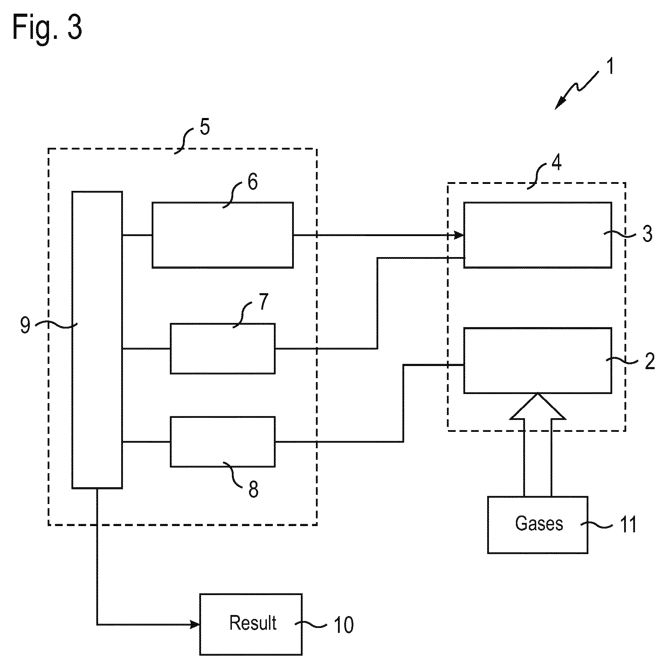

Figure 3 shows a single gas sensor comprising a MOX resistor 2 as a sensing element die, a heater 3, a signal conditioning circuitry 5 that comprises a heater control unit 6, a first measurement unit 7 for measuring the heater resistance, a second measurement unit 8 for measuring the MOX resistance, and a state machine 9 functioning as a system controller and an EEPROM memory (not shown). Thereby, the heater 3 is connected to a heater control unit 6 which is controllable or operable connected to the state machine 9. The heater control unit 6 may be part of the signal conditioning circuitry 5. The signal conditioning circuitry 5 also comprises a first measurement unit 7 for measuring the heater resistance, a second measurement unit 8 for measuring the MOX-resistor resistance and a state machine 9 as a system controller. The first measurement unit 7 is used for measuring the heater resistance in order to determine the thermal energy that is submitted to the MOX resistor 2 by the heater 3. The second measurement unit 8 is used for measuring the MOX-resistor 2 resistance in dependence of the temperature inputted by the heater 3 and of the present gases that interact with the surface of the metal oxide film of the MOX resistors 2. The state machine 9 works as a system controller which pretends to set the temperature to a pre-determined value in order to perform the inventive method for detecting and calculating concentrations based on the measured results.

It is proposed that the MOX resistor 2 as sensing element is set to a temperature and readings of the MOX sensor 2 are taken at short intervals, for example every 10msec or 50ms. In the next step the temperature for the MOX resistor 2 should be set to a higher temperature and the measurement cycle is repeated. The change of temperature can be repeated to improve discrimination of the gas composition and concentration. The results become a matrix that shows the reaction of the MOX resistor 2 to the atmosphere at different times and the rate of reactions. In the next step, the measured sensor data will be processed by an evaluation algorithm. The attributes that are offset shifts, within and amongst temperature settings, slope within a temperature setting. For example, a negative slope at 250°C and a positive slope at 350°C is a behavior that signals a presence of carbon monoxide.

**Reference Signs**

1 Single gas sensor
2 MOX resistor
3 heater
4 sensing element
5 signal conditioning circuitry, control circuitry
6 heater control unit
7 first measurement unit
8 second measurement unit
9 state machine as a system controller
10 result
11 measurement gas / gas composition

**Claims**

1. A single gas sensor (1) for sensing different gases comprising a MOX resistor (2) as sensing element (4), a heater (3), and a signal conditioning circuitry (5), whereas said signal conditioning circuitry (5) comprises a heater control unit (6) connected to the heater 3), a first measurement unit (7) for measuring the heater resistance, a second measurement unit (8) for measuring the MOX-resistor (2) resistance and a state machine as a system controller (9).

2. A single gas sensor for sensing different gases according to claim 1, wherein said signal conditioning circuitry is an Application Specific Integrated Circuit (ASIC).

3. A single gas sensor (1) for sensing different gases according to claim 1, wherein said heater control unit (6) is controllable connected to the heater (3) for adjusting a pre-defined temperature regime to the heater (3).

4. A single gas sensor (1) for sensing different gases according to claim 3, wherein said temperature regime is adjustable form 100°C to 550°C.

5. A single gas sensor (1) for sensing different gases according to claim 1, wherein the MOX resistor (2) and the heater (3) are stacked on a silicon die on a thin membrane or on a ceramic substrate with a low thermal mass.

6.  A single gas sensor (1) for sensing different gases according to claim 1, wherein said state machine (9) is controllable connected to the heater control unit (6), to the first measurement unit (7) for measuring the heater resistance, and to the second measurement unit (8) for measuring the MOX-resistor (2) resistance.

7.  A method for sensing different gases using the single gas sensor (1) according to claims 1 to 6, wherein the method comprising the following steps:

    - setting the heater (3) and the MOX resistor (2) to a first temperature;
    - measuring the MOX resistance at time intervals pre-determined via the state machine (9) for the first temperature;
    - setting the heater (3) and the MOX resistor (2) to a second temperature;
    - measuring the MOX resistance at time intervals pre-determined via the state machine (9) for the second temperature;
    - processing measured data and identifying a gas composition and calculating a concentration of different gases according to a determined temperature-resistance profile.

8.  A method for sensing different gases according to claim 7, wherein the second temperature is higher than the first temperature, whereas the temperature vary from 100°C to 550°C.

9.  A method for sensing different gases according to claim 7, wherein the time intervals for measuring is set to a sampling rate in excess of 1000/s.

10. A method for sensing different gases according to claim 7, wherein the method capturing the transient response which varies independently for different gases.

11. A single gas sensor (1) according to the former claims are used for indicating a gas composition and concentration for carbon monoxide, breathe alcohol and/or volatile organic compounds (VOC).

## Fig. 1

## Fig. 2

Fig. 3

Europäisches
Patentamt

European
Patent Office

Office européen
des brevets

# EUROPEAN SEARCH REPORT

Application Number

EP 16 18 9400

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 725 350 A2 (UNITRONIC AG [DE]) 30 April 2014 (2014-04-30) | 1,3,4, 6-9,11 | INV. G01N27/12 |
| Y | * paragraphs [0007], [0008], [0019] - [0027], [0031], [0032], [0038]; figure 3 * | 2,5,10 | ADD. G01N33/497 |
| X | EP 0 829 718 A1 (KOHLER HEINZ PROF DR [DE]) 18 March 1998 (1998-03-18) * column 1, lines 3-7, 40-43 * * column 4, lines 17-37 * * column 6, lines 14-30 * * column 6, line 45 - column 8, line 53 * | 1,7 | |
| Y | WO 2015/071337 A1 (LFOUNDRY S R L [IT]) 21 May 2015 (2015-05-21) * page 3, line 17 - page 4, line 16 * | 2 | |
| Y | US 2015/021716 A1 (LEE DAE-SIK [KR] ET AL) 22 January 2015 (2015-01-22) * figure 1 * | 5 | |
| Y | US 2014/105790 A1 (GAUDON ALAIN [FR] ET AL) 17 April 2014 (2014-04-17) * figure 2 * | 5 | TECHNICAL FIELDS SEARCHED (IPC) G01N |
| Y | US 2009/312954 A1 (UTRIAINEN MIKKO [FI]) 17 December 2009 (2009-12-17) * paragraph [0059]; figures 1, 8 * | 5,10 | |
| Y | US 6 902 701 B1 (HUGHES ROBERT C [US] ET AL) 7 June 2005 (2005-06-07) * column 13, lines 13-39; figure 3 * | 10 | |

The present search report has been drawn up for all claims

| Place of search | Date of completion of the search | Examiner |
|---|---|---|
| Munich | 9 February 2017 | Knoll, Stephan |

EPO FORM 1503 03.82 (P04C01)

## ANNEX TO THE EUROPEAN SEARCH REPORT
## ON EUROPEAN PATENT APPLICATION NO.

EP 16 18 9400

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2017

| Patent document cited in search report | | Publication date | Patent family member(s) | | Publication date |
|---|---|---|---|---|---|
| EP 2725350 | A2 | 30-04-2014 | DE 102012110095 | A1 | 24-04-2014 |
| | | | EP 2725350 | A2 | 30-04-2014 |
| EP 0829718 | A1 | 18-03-1998 | AT 264503 | T | 15-04-2004 |
| | | | DE 19639072 | A1 | 12-03-1998 |
| | | | EP 0829718 | A1 | 18-03-1998 |
| WO 2015071337 | A1 | 21-05-2015 | CN 106030297 | A | 12-10-2016 |
| | | | EP 3069130 | A1 | 21-09-2016 |
| | | | JP 2016537654 | A | 01-12-2016 |
| | | | KR 20160106567 | A | 12-09-2016 |
| | | | US 2016290946 | A1 | 06-10-2016 |
| | | | WO 2015071337 | A1 | 21-05-2015 |
| US 2015021716 | A1 | 22-01-2015 | KR 20150010473 | A | 28-01-2015 |
| | | | US 2015021716 | A1 | 22-01-2015 |
| US 2014105790 | A1 | 17-04-2014 | CN 103718031 | A | 09-04-2014 |
| | | | CN 105784787 | A | 20-07-2016 |
| | | | EP 2533037 | A1 | 12-12-2012 |
| | | | EP 2718705 | A1 | 16-04-2014 |
| | | | JP 2014519042 | A | 07-08-2014 |
| | | | KR 20140074269 | A | 17-06-2014 |
| | | | US 2014105790 | A1 | 17-04-2014 |
| | | | WO 2012168444 | A1 | 13-12-2012 |
| US 2009312954 | A1 | 17-12-2009 | EP 2013612 | A1 | 14-01-2009 |
| | | | FI 20060389 | A | 22-10-2007 |
| | | | US 2009312954 | A1 | 17-12-2009 |
| | | | WO 2007122287 | A1 | 01-11-2007 |
| US 6902701 | B1 | 07-06-2005 | NONE | | |

EPO FORM P0459

For more details about this annex : see Official Journal of the European Patent Office, No. 12/82